# EUROPEAN PATENT SPECIFICATION

(11) **EP 0 831 797 B1**
(45) Date of publication and mention of the grant of the patent: **07.03.2007**
(21) Application number: 96916256.9
(22) Date of filing: 07.06.1996
(51) Int. Cl.: A61K 31/045, A61P 27/00

(54) **PHARMACEUTICALLY ACTIVE CAROTENOIDS**
PHARMAZEUTISCH AKTIVE KAROTENOIDE
CAROTENOIDES PHARMACEUTIQUEMENT ACTIFS

(30) Priority: 07.06.1995 US 487627; 28.02.1996 GB 9604221
(43) Date of publication of application: 01.04.1998
(73) Proprietor: The Howard Foundation, Cambridge, Cambridgeshire CB2 5JY (GB)
(72) Inventor: HOWARD, Alan, Norman Whitehill House, Cambridgeshire CB2 5JY (GB); LANDRUM, John, Thomas Dept. of Chemistry, Miami, FL 33199 (US); BONE, Richard, Andrew Dept. of Physics, Miami, FL 33199 (US)
(74) Representative: Lipscombe, Martin John
(86) International application number: PCT/GB1996/001368
(87) International publication number: WO 1996/040092

(56) References cited:
- EP-A- 0 385 335
- EP-A- 0 672 655
- WO-A-91/03571
- WO-A-96/19215
- DE-A- 4 020 874
- US-A- 4 048 203
- US-A- 5 310 764
- US-A- 5 457 135
- INVESTIGATIVE OPHTHALMOLOGY & VISUAL SCIENCE, vol. 36, no. 4(suppl.), 15 March 1995, page s892 XP002016871 J.T.LANDRUM ET AL.: "MACULAR PIGMENT STEREOMERS IN INDIVIDUAL EYES: A COMPARISON BETWEEN NORMALS AND THOSE WITH AGE-RELATED MACULAR DEGENERATION (AMD)"
- J.AM.OSTEOPATH.ASSOC., vol. 95, no. 1, January 1995, page 26 XP000603675 N.N.: "Do antioxidants prevent or retard the onset of AMD?"
- JAMA, vol. 272, no. 18, 9 November 1994, pages 1413-1420, XP000603671 J.M.SEDDON ET AL.: "Dietary Carotenoids, Vitamins A, C, and E, and Advanced Age-Related Macular Degeneration"
- AM.J.CLIN.NUTR., vol. 62, no. 6 suppl., December 1995, pages 1448S-1461S, XP000603641 D.M.SNODDERLY: "Evidence for protection against age-related macular degeneration by carotenoids and antioxidant vitamins"
- EXS, vol. 62, 1992, pages 280-298, XP000603685 W.SCHALCH: "Carotenoids in the retina - A review of their possible role in preventing or limiting damage caused by light and oxygen"
- ARCH.OPHTHALMOL., vol. 113, no. 12, December 1995, pages 1518-1523, XP000603644 J.A.MARES-PERLMAN ET AL.: "Serum Antioxidants and Age-Related Macular Degeneration in a Population-Based Case-Control Study"
- NUTR.REV., vol. 38, no. 11, November 1980, pages 384-386, XP000603663 N.N.: "THE EFFECT OF A DIETARY LACK OF XANTHOPHYLL ON THE EYE OF THE MONKEY"
- DIALOG(R)FILE 35: DISSERTATION ABSTRACTS ONLINE, ACCESSION NUMBER 01465553, XP002016872 E.J.N. ROUSSEAU: "CAROTENOIDS AND OTHER DIETARY ANTIOXIDANTS IN FREE RADICAL RESEARCH: PROTECTION OF HUMAN RETINA HOMOGENATE AGAINST PHOTOCHEMICAL AND METAL-INDUCED LIPID AUTOXIDATION"
- BONE R.A. ET AL: 'DISTRIBUTION OF THE MACULAR CAROTENOIDS ZEAXANTHIN, MESO-ZEAXANTHIN AND LUTEIN IN THE HUMAN RETINA' IOVS vol. 34, no. 4, 15 March 1993, US, page 1199, XP008045873
- BONE R.A. ET AL IOVS vol. 42, no. 1, 2001, pages 235 - 240

## Description

The invention relates to the use of meso-zeaxanthin which increases the deposition of macular pigment in the human eye. The invention is particularly but not exclusively concerned with pharmaceutical compositions containing meso-zeaxanthin for use in the treatment by therapy or prophylaxis of disease of the macular and in particular age-related macular degeneration (AMD).

The macula is the anatomical region of the retina which in man is responsible for central vision. Centered on the fovea, where the visual axis meets the retina, it extends radially outwards to a distance of about 2.75 mm (Davson, 1990). The macula is divided into the inner macula and the outer macula. The inner macula extends radially out to a distance of 1.5 mm while the outer macula is defined by the surrounding annular ring. The central part of the macula is easily recognisable because of its yellow coloration which results from the presence of macular pigment.

Despite its small size the macula is endowed with the highest degree of visual acuity. It is therefore not surprising that considerable effort is devoted to understanding and, when possible, treating diseases which disrupt the normal functioning of the macula. One such disease is age-related macular degeneration (AMD) which occurs in about 20% of the population above the age of 65 and is the leading cause of visual impairment in the USA and UK. AMD has up to the present been an irreversible condition.

Pooled extracts of the macular pigment were found by Wald (1945) to have a carotenoid-like absorption spectrum which appeared to match that of lutein. Further work in the 1980's demonstrated that it consisted of lutein and zeaxanthin (Bone *et al* 1985).

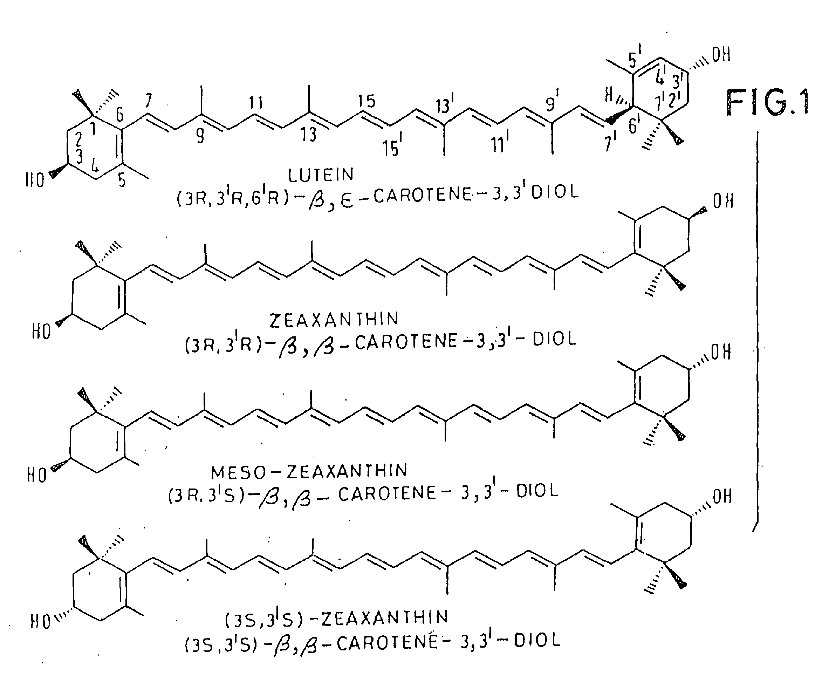

More recent work (Bone *et al* 1993) has shown that the zeaxanthin component found in the human retina is itself composed of all three of the possible stereoisomers. Figure 1 shows the stereochemical structures of the macular pigment components. The 3' hydroxy groups on lutein and meso-zeaxanthin have the same absolute configuration making interconversion possible by a movement of the 4'-5' double bond (lutein) to the 5'-6' position (meso-zeaxanthin). Of the three stereoisomers, SSZ is present only as a relatively small component. RRZ is of dietary origin whereas RSZ (or meso-zeaxanthin) is not common in the diet and has yet to be detected in human serum. It has been suggested that the presence of RSZ may be the result of isomerization of lutein to RSZ by an enzyme.

WO 91/03571 discloses a method of preparing zeaxanthin.

The function of the macular pigment has not been unequivocally determined. It has been proposed that one function may be to reduce the adverse effect of chromatic aberration in the ocular media thereby increasing acuity (Walls 1967: Reading and Weale 1974). Currently, a more generally held view is that the pigment probably acts in a protective capacity against the damaging effects of blue light (Dicthburn 1973, Kirshfeld 1982, Bone *et al* 1984) which can induce the formation of reactive free radicals within the retina and the formation of such species may be greatly reduced in individuals having a high level of macular pigmentation. The macular pigment may also serve passively as a filter and shield sensitive tissues from harmful excessive blue light.

AMD is a disease which develops gradually over a period of many years with loss of sight being the ultimate result. The damaged tissue has an unusually high lipid content which it has been suggested oxidises to form lipofuscin, a fluorescent product of lipid oxidation. It has been postulated that exposure of the retina to excessive blue light may increase the rate of lipofuscin formation (Feeney-Burns *et al* 1990, Gottsch *et al* 1990).

To date, little is known about the factors which influence the uptake of carotenoids into the macula and there is no effective cure or prevention of AMD.

The studies of plasma carotenoids in case control studies of AMD have been equivocal. In the Beaver Dam eye study *(*Mares*-*Perlman *et al* 1995), no differences were observed in 167 cases and 167 controls in serum including lutein or zeaxanthin. In the Eye Disease Case Control Study Group (1993) results of 421 cases and 615 controls were reported.

People with serum carotenoid levels in the medium to high group had one half to one third risk of AMD. All the carotenoids measured including lutein, zeaxanthin, beta carotene, alpha carotene and cryptoxanthin were implicated. In a further publication (Seddon *et al,* 1994), these authors found that the consumption of lutein and zeaxanthin (which are primarily obtained from dark green leafy vegetables) were most strongly associated with a reduced risk for AMD. However, some people with a high consumption of green vegetables still suffered from AMD. In addition, Seddon *et al* (1995) investigated the relationship between the risk of developing AMD and the dietary intake of carotenoids and vitamins A, C and E. A higher dietary intake of carotenoids was associated with a lower risk of AMD.

Schalch (1992) investigated the idea that the concentration of dietary carotenoids in the macula is not accidental, but their presence may prevent or limit damage due to their physiochemical properties and a capacity to quench oxygen free radicals and singlet oxygen, which are generated in the retina as a consequence of the simultaneous presence of light and oxygen.

WO96/19215 discloses a method for the prevention or treatment of arteriosclerosis in a mammal, wherein the method comprises administering an effective amount of a natural tocopherol and a natural carotene to prevent or treat arteriosclerosis in a mammal in need of such prevention or treatment.

The authors of DE4020874 (and the corresponding US patent 5,290,605) disclose a nutritional soft drink for the protection against the danger of exposure to UV light, wherein the drink comprises carotenoids, optionally together with vitamin C and/or vitamin E and/or other physiologically acceptable antioxidants.

In an abstract published in the March 1995 issue of Investigative Ophthalmology and Visual Science (36, suppl, 892), the carotenoid analysis of 8 normal eyes and 8 eyes from patients with AMD was reported. The results suggested a positive correlation existed between lowered macular pigment and the prevalence of AMD, but recommended that caution should be exercised in this interpretation because the reduced macular pigment could be a result, rather than a cause, of the disease. When the subject-matter of the above mentioned abstract was submitted for publication to a peer-reviewed journal, the referees recommended rejection because the number of samples analysed was too small. Further results were therefore necessary before any conclusion could be made on the possible preventative role of lutein/zeaxanthin in AMD.

It is the object of the present invention to increase macular pigment and to prevent or cure AMD by the administration of a pharmaceutical composition comprising meso-zeaxanthin in combination with a pharmaceutical carrier or diluent.

It is a further object of the invention to provide the use of meso-zeaxanthin in the manufacture of a medicament for use in the treatment of macula depreciation of yellow pigment in the macula of an eye of a human patient.

Within the above context we have now found surprisingly that by selecting a particular type of carotenoid, namely meso-zeaxanthin thereof, it is possible to increase the macular pigment in the human macula which could lead to the prevention and/or treatment of AMD in those people at risk or with the disease.

Moreover the effective dose is rather surprisingly greater than that which is normally achieved by the intake of zeaxanthin in rich green vegetables. While it could be suspected that since the macula contains zeaxanthin, the administration of zeaxanthin in quantities similar to that occurring in green vegetables would raise the concentration of macular pigment, it has been found rather surprisingly that when the carotenoid is given orally in a concentrated form the amount required to be effective in the short term is considerably greater than expected.

Furthermore, it has been found that in a sufficiently large enough sample to warrant conclusions, the lutein/zeaxanthin content of the retinas of eyes from people with AMD was 30 % less than people with normal eyes.

Accordingly, the present invention in one aspect provides meso-zeaxanthin for use as a pharmaceutical or food supplement, particularly in the elevation of macular pigment and the prevention or management of age-related macular degeneration. Generally speaking, the active agent (i.e. meso-zeaxanthin) may be used in the total dosage regime of up to 100mg per day typically 10-50mg per day with an optimum dosage of 30mg per day.

The dose depends on the time of administration. When the macular is depleted of macular pigment, a high dose (circa 30mg/day) is normally used. Uses according to the invention are principally directed to high dosage of the patient (i.e. amounts of 10mg/day or higher) and are especially concerned in preferred embodiments to achieve serum levels of the carotenoid of at least 0.7 or 0.8mm/ml.

During the initial period of administration, it is preferred to use a large dose of circa 30mg/day for several weeks. However, when a plateau is achieved in the concentration of macular pigment a maintenance dose of e.g. circa 7.5mg/day is preferable. The reason for this is that at the high dose, the skin turns yellow caused by the yellow colour of meso-zeaxanthin. This is an undesirable side-effect. Whilst it can be tolerated for a short time, a lower dose is preferable for maintenance since it is sufficient to maintain the level of macular pigment to a desirable level, and does not cause skin pigmentation.

A unit dosage form such as say a 750mg tablet or say an 800mg capsule to be used on a one-a-day basis may contain from 0.1 % to about 12.5 % by weight of zeaxanthin and other ingredients may comprise:

| | |
|---|---|
| Beta carotene | about 2 to about 20mg e.g. about 5mg |
| Lycopene | about 2 to about 20mg e.g. about 5mg |
| Vitamin A | about 400 to about 600 RE e.g. about 500 RE |
| Vitamin C | about 75 to about 250mg e.g. about 100mg |
| Vitamin E | about 50 to 250mg e.g. about 100mg |
| Selenium | about 80 to about 120mcg e.g. about 90mcg |
| Copper | about 2 to about 4mg e.g. about 3mg |
| Zinc | about 10 to about 20mg e.g. about 15mg |
| Manganese | about 2 to about 5mg e.g. about 4mg |
| Ubiquinone | about 10 to about 100mg e.g. about 50mg |
| (Coenzyme Q10) Carrier | about 150 to about 250mg e.g. about 175 or about 200mg |

Accordingly, it may be desirable to employ a high dose of meso-zeaxanthin followed by a lower dose when the macular pigment reaches a plateau. For those skilled in the art, macular pigmentation can be measured by a flicker photometer (see Example 2).

The composition of the invention is especially useful in increasing the macular pigment in the human macula and in the preventive treatment of age-related macular degeneration.

As will be seen from Figure 1 of the drawings, lutein and zeaxanthin are stereoisomers. Zeaxanthin diastereomers can exist in three different forms in nature, namely zeaxanthin (the 3R, 3'R form) meso-zeaxanthin (the 3R, 3'S form) and 3S, 3'S zeaxanthin.

Meso-zeaxanthin is an isomer which does not occur naturally (at least in any abundance) other than in the primate eye, and is thought to be synthesized in the eye by enzymatic conversion.

In carrying out the invention, there may be used a compound as defined in its free form.

The invention provides a pharmaceutical composition, which composition comprises meso-zeaxanthin together with a pharmaceutically accepted diluent or carrier.

Such a composition may be in bulk form, or more preferably, unit dosage form. Thus, for example, the composition may be formulated as a tablet, capsule, powder, solution or suspension.

Compositions in accordance with the invention may be prepared using the carotenoid active agent in accordance with conventional food supplement or pharmaceutical practice. The diluents, excipients or carriers which may be used are well known in the formulation art and the form chosen for any particular regimen will depend on the given context and the formulator's choice.

In carrying out the invention, the active carotenoid may be used together with other active agents. Amongst such other agents, there may be mentioned, for example, the following, namely another carotenoid such as lycopene or alpha, beta, gamma or delta carotene, or one or more of the following antioxidants, namely vitamin A, vitamin C, vitamin E (α-tocopherol and other active tocopherols), selenium, copper, zinc, manganese and ubiquinone (coenzyme Q10).

Use of a mixture containing a tocopherol such as α-tocopherol is especially preferred since it is believed that such a mixture affords a synergistic effect.

The carotenoids are partially destroyed in the gastrointestinal tract by oxidation. By adding vitamin E and/or vitamin C, this process is inhibited and more carotenoid is absorbed. The inhibitor may be included as part of a composition, as part of the invention or administered separately.

In addition to the above aspects, the invention includes the use of the carotenoid meso-zeaxanthin thereof, in the manufacture of a medicament for increasing the pigment in the macula of the human eye for treatment or prevention of age-related macular degeneration or other macular pigment depreciation malady.

Furthermore, the invention includes a process for the manufacture of a medicament for the above-mentioned purposes.

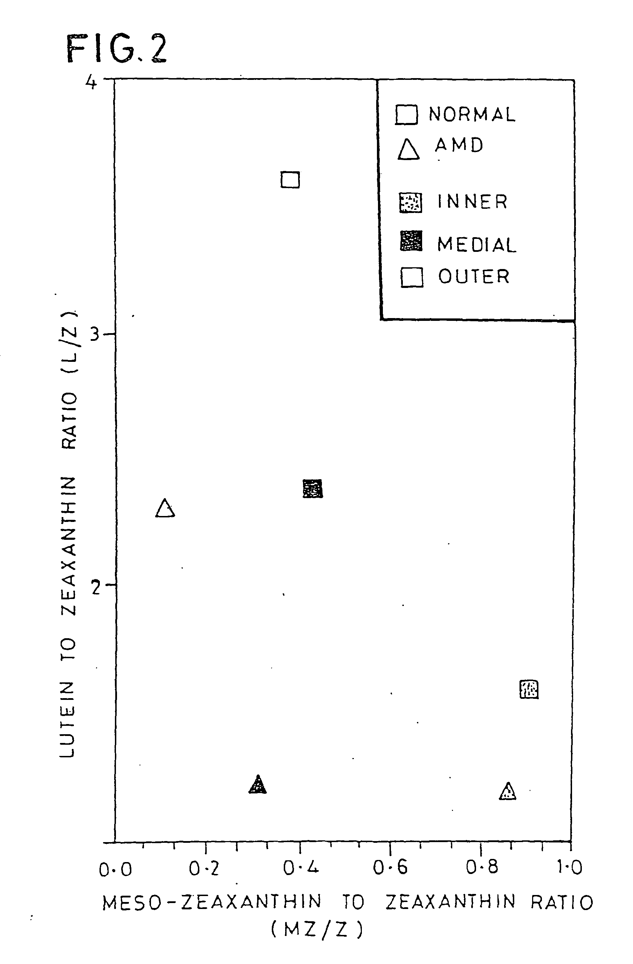

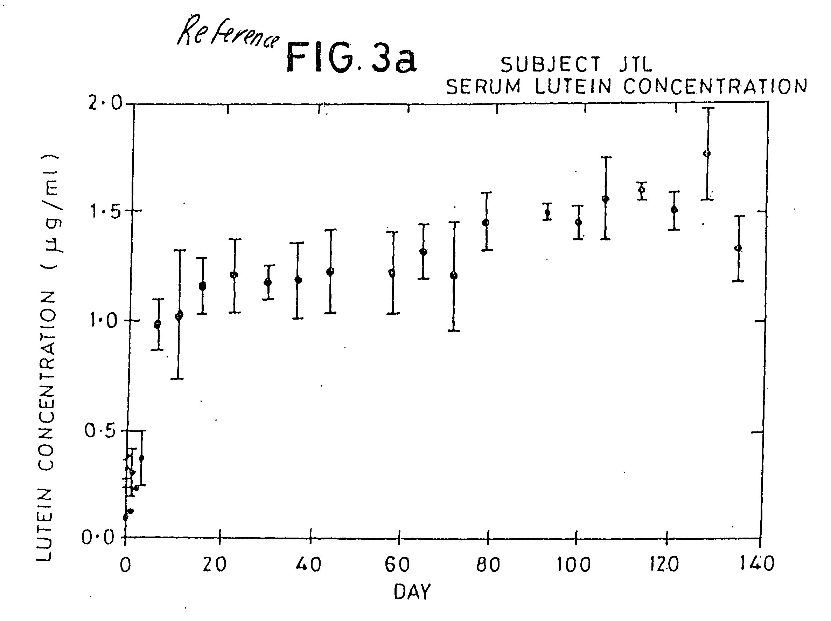

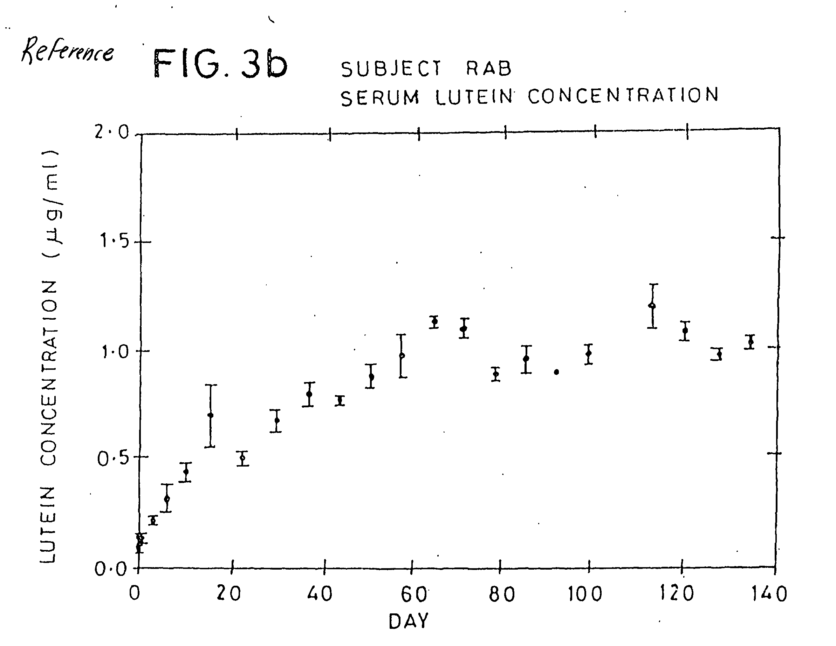

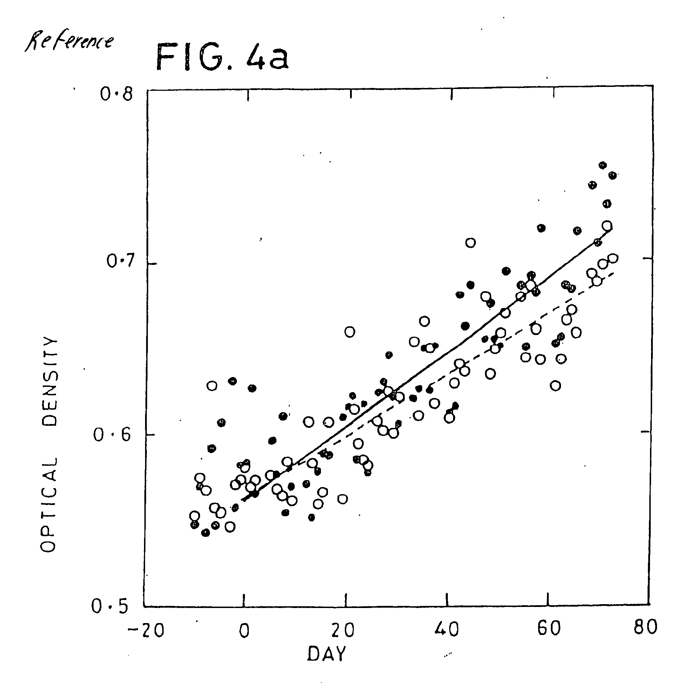

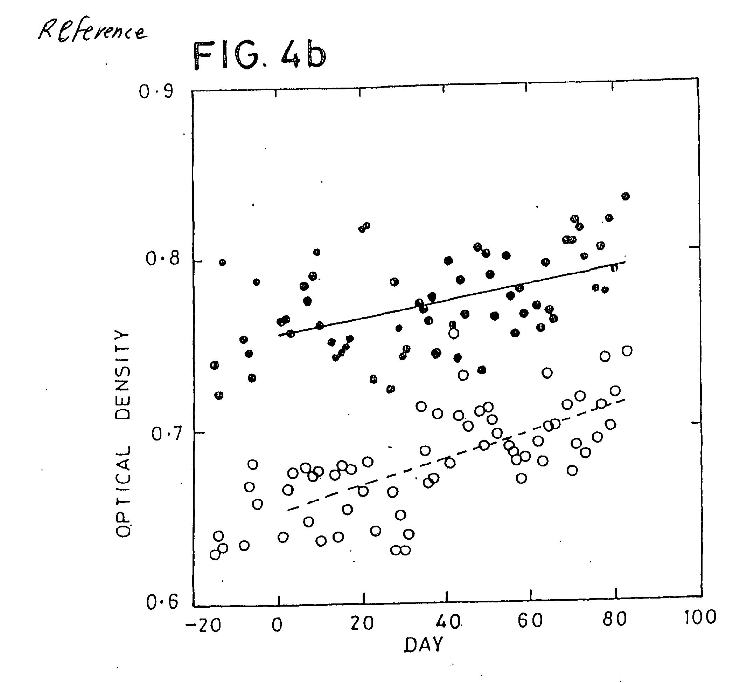

The following Examples are intended to illustrate the invention by way of example only. Reference in the Examples is made to Figures 2 to 4 of the drawings, wherein:
Figure 2 shows for both normal and AMD eyes the average L:Z ratio for each disc or annulus of retinal tissue plotted against the average MZ:Z ratio. The ratios of lutein to zeaxanthin and meso-zeaxanthin are consistently lower for AMD eyes as compared to normals.
Reference Figure 3a shows the time dependent increase in the serum lutein level of Subject JTL (Example 2). Error bars represent the standard deviations in the measurements. Day "0" represents the beginning of lutein supplementation.
Reference Figure 3b shows the time dependent increase in the serum lutein level of subject RAB (Example 2). Error bars represent the standard deviations in the measurements. Day "0" represents the beginning of lutein supplementation.
Reference Figure 4a shows the daily macular pigment optical density measurements for subject JTL (Example 2) from 7 days prior to the start (day "0") of the lutein supplementation through day 72. Left eye - solid circles; right eye - open circles. The solid line is the linear least squares fit to the left eye data and has a slope of 15.3 x 10⁻³ absorbance units per week. The dashed line is a fit to the right eye data and has a slope of 12.5 x 10⁻³ absorbance units per week.
Reference Figure 4b shows the daily macular pigment optical density measurements for subject RAB (Example 2) from 7 days prior to the start (day "0") of the lutein supplementation through day 83. Left eye - solid circles; right eye - open circles. The solid line is the linear least squares fit to the left eye data and has a slope of 3.1 x 10⁻³ absorbance units per week.

The dashed line is a fit to the right eye data and has a slope of 2.3 x 10⁻³ absorbance units per week.

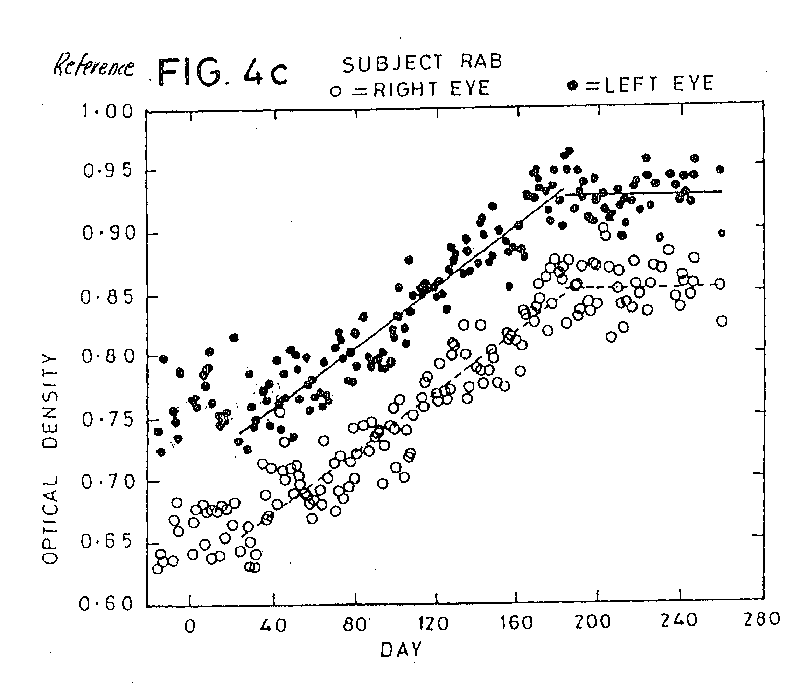

Reference Figure 4c shows daily macular pigment optical density measurements for the same subject as is the case in Figure 4b for a longer period of lutein administration which includes the period represented in Figure 4b.

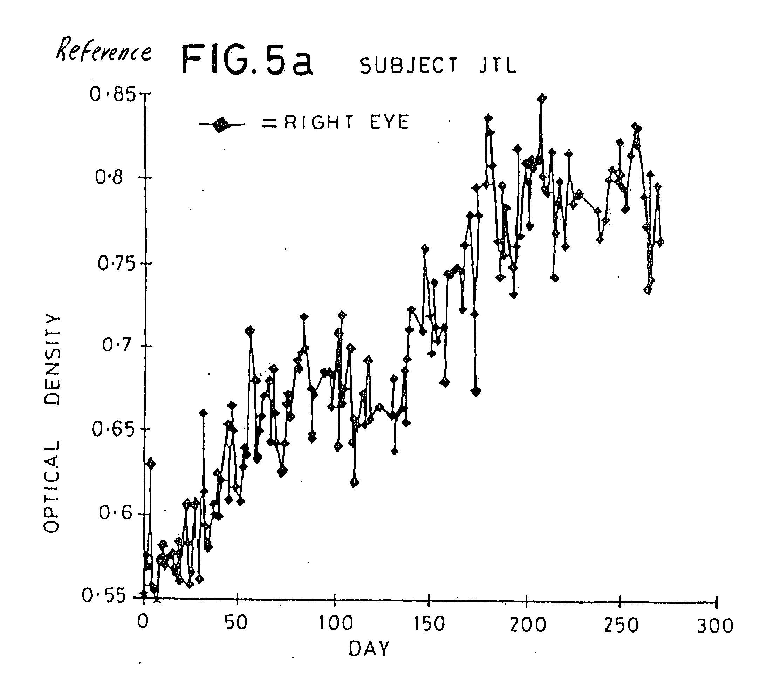

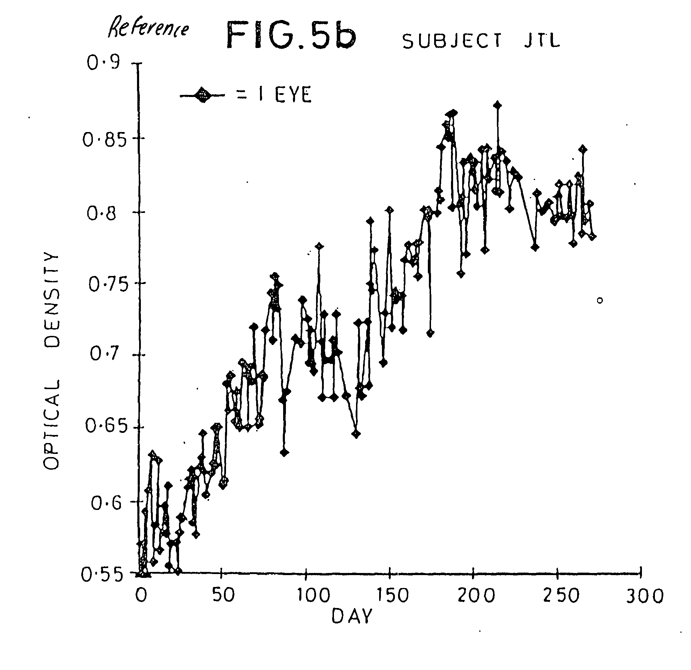

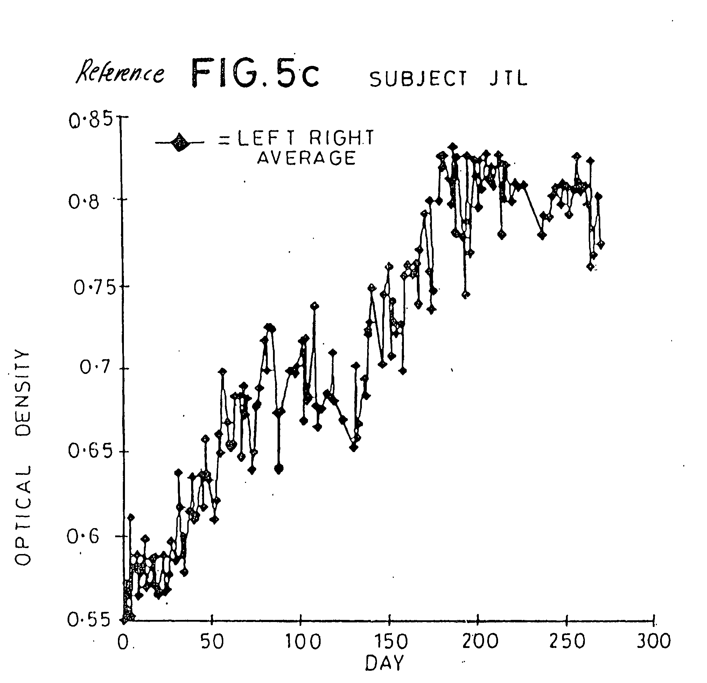

Reference Figures 5a, 5b and 5c show the daily macular pigment optical density measurements for the same subject as is the case in Figure 4a for a longer period of lutein administration which includes the period represented in Figure 4a, Figure 5a relating to the right eye of the subject, Figure 5b relating to the left eye and Figure 5c representing the L-R average.

### Example 1

### 1.1 Analysis of carotenoids in eyes

An HPLC analysis of retinas obtained from normal and AMD individuals was conducted using a sufficiently large sample to warrant conclusions on the importance of macular lutein and zeaxanthin in the prevention of AMD. The amount and distribution of the macular carotenoids, including the stereo isomers, were determined and compared for 15 normal and 22 AMD eyes in order to determine if there is evidence for or against the hypothesis that macular pigment protection from light exposure plays a significant role in reducing AMD.

For each normal and AMD eye, the neural retina was cut into a central disk and 2 concentric annuli using trephines of 3, 11 and 21 mm. To extract the carotenoids, the tissues were ground in ethanol/water (1:1) to which 10ng lutein monomethyl ester was added as an internal standard. Separation and quantitation of zeaxanthin and lutein fractions was by reversed-phase HPLC using Phenomenex column. Carbamate derivatives of individual stereomers of both zeaxanthin and lutein were separated on a normal-phase HPLC column using the methods of Ruttiman *et al* (1983) and Schiedt *et al* (1995), the results being plotted in Figure 2.

### 1.2 Results and Conclusions

As shown in the Table below, AMD eyes had on average approximately 70 % of the total carotenoid found in controls, a figure that was very consistent across the retina. Seventeen (77%) of the twenty two AMD eyes had total amounts of lutein and zeaxanthin in the central 3mm of the retina which were below the mean (5.9pmole/mm²) for the control group. For the two annuli, having outer diameters of 11 and 21mm respectively, 15 (68%) of the AMD group were found to be lower in total carotenoids than the corresponding regions in the control group.

The differences observed between the control and AMD eyes in the inner annuli were found to be statistically significant (on a one sided test p<0.05); the difference in the medial and outer annuli were found almost significant (p<0.1).

The relative distributions of carotenoids throughout the retina for normal and AMD eyes were found to be essentially the same. Both groups were characterised by a decrease in the quantity of meso-zeaxanthin and a relative increase in lutein with increasing distance from the fovea. The relative amounts of lutein and meso-zeaxanthin as compared to zeaxanthin are consistently lower in the AMD retinas as compared to normal retinas.

| TOTAL CAROTENOID/UNIT AREA (pmoles/mm²) | | | | |
|---|---|---|---|---|
| Donor# | | INNER (7.1 mm²) | MEDIAL (93 mm²) | OUTER (343 mm²) |
| | 1 | 12.8 | 0.88 | 0.19 |
| | 2 | 10.5 | 0.51 | 0.10 |
| | 3 | 10.4 | 0.89 | 0.18 |
| | 4 | 9.3 | 1.35 | 0.36 |
| | 5 | 8.4 | 0.38 | 0.07 |
| | 6 | 5.8 | 0.19 | 0.06 |
| | 7 | 5.3 | 0.23 | 0.43 |
| CONTROL | 8 | 5.1 | 0.48 | 0.21 |
| EYES | 6 | 4.7 | 0.15 | 0.05 |
| | 9 | 4.6 | 0.21 | 0.06 |
| | 9 | 4.3 | 0.18 | 0.05 |
| | 10 | 2.5 | 0.07 | 0.03 |
| | 10 | 2.2 | 0.08 | 0.03 |
| | 11 | 2.0 | 0.26 | 0.20 |
| | 12 | 1.0 | 0.09 | 0.02 |

| Control average ± sd | | 5.9 ± 3.4 | 0.04 ± 0.36 | 0.14 ± 0.12 |
|---|---|---|---|---|
| | 13 | 9.5 | 0.47 | 0.19 |
| | 13 | 9.4 | 0.78 | 0.23 |
| | 14 | 8.4 | 0.30 | 0.12 |
| | 14 | 7.7 | 0.56 | 0.13 |
| | 15 | 6.7 | 0.17 | 0.09 |
| | 16 | 5.7 | 0.24 | 0.08 |
| | 17 | 4.8 | 0.47 | 0.15 |
| | 18 | 4.5 | 0.34 | 0.07 |
| | 16 | 4.5 | 0.20 | 0.06 |
| | 19 | 4.5 | 0.14 | 0.05 |
| | 17 | 4.0 | 0.24 | 0.11 |
| | 19 | 3.8 | 0.05 | 0.09 |
| | 20 | 3.4 | 0.45 | 0.16 |
| | 21 | 3.4 | 0.20 | 0.09 |
| | 20 | 2.4 | 0.46 | 0.13 |
| | 22 | 2.3 | 0.13 | 0.05 |
| | 22 | 1.9 | 0.11 | 0.06 |
| | 23 | 1.2 | 0.03 | 0.02 |
| | 1 | 0.71 | 0.47 | 0.19 |
| | 23 | 0.46 | 0.03 | 0.02 |
| | 24 | 0.43 | 0.10 | 0.03 |
| | 24 | 0.32 | 0.07 | 0.02 |

### Reference Example 2 - Uptake of lutein in human adults

### (Outside the scope of the invention)

### 2.1 Serum Uptake

A trial was conducted to determine if dietary supplementation with lutein and zeaxanthin effectively can change the pigment levels in the macula.

The optical density of the macula pigment was measured for each subject using the method of flicker photometry (Bone and Sparrock 1971; Bone *et al* 1992). The concentration of pigment in the macula is proportioned to its optical density and the actual amount of pigment was assumed to be proportional to concentration. Thus, optical density was taken as a measure of the total amount of pigment.

Serum lutein and zeaxanthin was measured by conventional HPLC.

Two healthy adult males (of age/weight 42 year/60kg and 51 years/61kg) ingested the equivalent of 30mg of lutein per day in the form of lutein esters (source: marigold flowers) suspended in 2ml of canola oil. This was continued over a period of 138 days and then the dose of lutein was discontinued. Chemical analysis has shown that the product contains approximately 97% lutein and 3% zeaxanthin. Fasting serum lutein/zeaxanthin levels of both individuals were determined by conventional HPLC on the morning of the first dose as a measure of base line. Blood samples were drawn at 2-3 hour intervals thoughout the first day for both subjects and then daily for the next three days. Following the first week of supplementation, blood samples were drawn weekly. Blood was collected into a standard Vacutainer serum separator tube containing no anticoagulent. After allowing about 30 min for coagulation, the sample was centrifuged for 10 minutes and the serum removed by pipette. Serum samples were stored at -20°C prior to analysis. Carotenoids were extracted from the serum by a minor modification of widely used methods (Guiliano *et al,* 1993; Handelmann *et al,* 1992). 200µl aliquots of serum were diluted with 2ml of 50% ethanol/water to ensure precipitation of protein components. 20µl of an internal standard, monohexyl lutein ether, containing about 90 ng, was added to the solution at this point for quantification of the carotenoids by HPLC. This solution was extracted 3 times with 2ml portions of hexane by vortexing the sample for 1 min followed by centrifuging for 5 minutes and pipetting off the hexane layer. The three portions of hexane were dried under a stream of nitrogen gas and stored under nitrogen at -20°C until analysis was completed.

Serum extracts were dissolved in 40 µl of ethanol prior to injection. Samples were vigorously agitated on a vortex mixer for 1 min to ensure dissolution of the sample. Two replicate analyses were carried out using 20µl aliquots. Serum carotenoids were eluted at a flow rate of 1 ml/min through a 15cm x 4.6mm Adsorbosphere ODS 3 µm HS column (Alltech) coupled to a 25 cm x 4.6 mm Spherisorb ODS 5µm column (Keystone Scientific) with detection of the carotenoids at 451 nm.

Figures 3a and 3b show the increase in serum lutein concentration in the two subjects during the time course of the supplementation experiment. The concentration of lutein in both subjects increased by a factor of about 10 times within the first week and remained high thereafter.

### 2.2 Macular Uptake

The optical density of the macular pigment was measured for each subject using the method of heterochomatic flicker photometry (Bone and Sparrock, 1971; Bone *et al,* 1992). The concentration of pigment in the macula is proportional to its optical density and the actual amount of pigment was assumed to be proportional to concentration. Thus optical density was taken to be a measure of the total amount of pigment.

In the flicker method, a small visual stimulus is presented to the eye which alternates in wavelength between 460 nm, the peak absorbance wavelength of the macular pigment, and 540 nm where pigment absorbance is zero (Bone *et al,* 1992). Above a certain frequency, colour fusion occurs but the stimulus continues to flicker. At a higher frequency, a critical condition can be reached where flicker can be eliminated only if the two wavelength components are matched in luminance. If the stimulus is viewed peripherally, so that the image falls outside the macula, neither wavelength is attenuated by the macular pigment. However, if the stimulus is viewed centrally, the intensity of the 460 nm light must be increased to compensate for absorption by the macular pigment in order to achieve a luminance match. Thus it is possible to determine the optical density of a subject's macular pigment at the peak wavelength, or indeed any other wavelength.

The validity of this technique depends on the relative spectral response of the receptors being the same in the central and peripheral locations used. The flicker, which the subject seeks to eliminate, is one of luminance and, assuming phototopic conditions, luminance is most likely due to an additive response from the long and middle wavelength sensitive cones (Guth *et al,* 1980). There is evidence that these two cone types are present in equal ratios in the two locations used (Wooten and Wald, 1973). The short wavelength cones, whose relative abundances differ between the two locations, are generally not assumed to contribute to luminance (Guth *et al,* 1980), though others, using flicker techniques, have sought to eliminate their participation (Pease *et al,* 1987; Werner *et al,* 1987; Hammond *et al,* 1995). The ultimate justification for our procedure is to be found in the accurate reproduction of the macular pigment absorbance spectrum which it generates (Bone *et al,* 1992).

The apparatus consisted of a two-channel Maxwellian view system based on a single light source, a 75 W xenon arc lamp. The wavelengths of the two channels were determined by 460 nm and 540 nm interference filters respectively, having half-widths of 7 and 9 nm. The channels were combined by a rotating semicircular mirror, and a circular aperture in a white screen provided a 1.5° diameter stimulus. Cross-hairs facilitated central fixation of the stimulus. The screen was 18° in diameter and was illuminated with white light from the same source. The illuminance of the screen was adjusted to provide the same retinal illuminance of 4 log Td as the stimulus. This was considered to be sufficiently high to minimise problems associated with rod intrusion which could otherwise differentially affect measurements in the macula and peripheral retina (Wyszcki and Stiles, 1982). A small red LED was located 8° above the centre of the stimulus to provide a fixation mark for peripheral viewing of the stimulus. The intensity of the 460 nm channel was adjustable by the subject through a neutral density, compensated wedge whose setting could be recorded by a push-button. The flicker frequency was also under the subject's control via a potentiometer. An adjustable dental impression bite ensured accurate and steady positioning of the subject's eye relative to the exit pupil.

The flicker frequency was set to a pre-determined value which, for central viewing by the subject, would allow flicker to be eliminated only over a very small range of wedge settings. This frequency was in the 25 to 35 Hz range. Having set the wedge to meet the no-flicker condition, the subject adapted to the viewing conditions by fixating with one eye on the stimulus cross-hairs for two minutes. The subject's other eye was occluded by an eye-patch. At the end of this period, the subject proceeded to make a series of 10 to 15 wedge settings, attempting to obtain the centre of the no-flicker range. The wedge was randomly offset after each setting. On occasions, the subject could not eliminate flicker entirely but instead sought a condition of minimum flicker. This was followed by another series of 10 to 15 settings while fixating on the LED, the frequency having been reduced to 12 to 16 Hz in order to reduce the range of no-flicker. The whole procedure was then repeated for the subject's other eye. The optical density of the macular pigment of the subject was measured daily for a period of one week prior to the commencement of lutein supplementation, and daily thereafter.

Figures 4a and 4b show the absorbency of the macular pigment during the time course of the experiments in the two subjects. An increase in the macular pigment level of subject JTL was first observable on the 14^{th} day of supplementation. This subject had macular pigment levels in both eyes that were experimentally determined by repeated measurements to be equal (±2 % ) the initial values of 0.57 and 0.58 being determined by averaging 15 measurements obtained over a 17 day time period. Comparison of these values with the average of 15 measurements obtained over the 18 day period at the end of the experiment gave values of 0.67 and 0.70 for the right and left eye, respectively, showing that the increase in optical density of the macular pigment is highly significant p<0.0005 for both the right and left eye, based on a one sided t test. After discontinuing the dose of lutein at day 138, optical density continued to rise until about day 180 and then reached a plateau.

For subject RAB, right and left eyes were found to have significantly different macular pigment density. The initial mean value for the right eye was 0.66 while that of the left eye was 0.76. This corresponded to a difference of 15% between the subject's left and right eyes. The increase in macular pigment determined by comparing the initial averages for each eye and the final average (0.70 right, 0.79 left) was found to be highly significant (p<0.001).

After discontinuing the dose at day 138, the optical density continued to rise in both eyes until day 200 and then reached a plateau. After several weeks of administration of lutein, the palms of the hands of each subject turned a noticeable yellow colour. This condition is similar to that induced by beta-carotene at the same dose.

Macular pigmentation increase was shown to be a slow process, despite the high plasma lutein levels. This may be partly due to the need for lutein to diffuse into the avascular macular region of the retina.

The trial established a relationship between the increased serum levels of lutein and corresponding increases in the concentration of lutein in the macula of the human eye. Long term lutein supplement of individuals having low levels of macular pigmentation could result in a significant increase in the level of pigmentation within the macula.

Our data suggests that macular pigmentation does function to protect the retina. An increased rate of photo oxidation might accompany lower macular pigment levels in some individuals and could contribute to a more rapid build up of pathological lesions associated with AMD.

### Reference Example 3

A capsule was prepared using the following ingredients by simple admixture and routine encapsulations:

| Ingredients per capsule | Label Claim | mg per Capsule |
|---|---|---|
| Lutein Ester | 30mg Lutein | 200 |
| Lecithin | | 50 |
| Soya Bean oil | | 200 |

One capsule per day/after a meal is recommended.
In the above example, lutein ester can be replaced by a mixture of isomers of zeaxanthin (normal zeaxanthin, meso zeaxanthin and 3S3'S zeaxanthin). Use of meso-zeaxanthin would place the resulting capsule within the scope of the present invention.

### Reference Example 4

A capsule was prepared using the following ingredients by simple admixture and routine encapsulation:

| Ingredients per capsule | Label Claim | mg per Caps |
|---|---|---|
| Lutein Ester | 10mg Lutein | 75 |
| Zeaxanthin Ester | 10mg zeaxanthin | 75 |
| Lecithin | | 25 |
| Soya Bean Oil | | 100 |

The above is a mixture of 50% each carotenoid. In the above capsule, zeaxanthin could represent all its isomers (zeaxanthin, meso zeaxanthin and 3S 3's zeaxanthin). Use of meso-zeaxanthin would place the resulting capsule within the scope of the present invention.

### Reference Example 5

### (Outside the Scope of the Invention)

A capsule was prepared using the following ingredients by simple admixture and routine encapsulation:

| Ingredients per capsule | Label Claim | mg per Caps |
|---|---|---|
| Vitamin C (ascorbic Acid) | 150mg | 160 |
| a-tocopheral | 100mg | 110 |
| Lutein Ester | 15mg Lutein | 90 |
| Lecithin | | 25 |
| Soya Bean Oil | | 75 |

A suitable daily dose for treatment AMD would be two capsules daily.

### Reference Example 6

### (Outside the Scope of the Invention)

The procedure of Example 7 was repeated except that 30mg of Coenzyme Q 10 was included in the mixture.

### Reference Example 7

### (Outside the Scope of the Invention)

A size 12 oval capsule of nominally 800mg weight was prepared from the following ingredients by simple admixture and routine encapsulation:

| Ingredients per capsule | Label Claim | mg per Caps |
|---|---|---|
| Vitamin A Palmitate 1500 iu/gm | 500 RE | 1.277 |
| Carotene Oil | 15mg BC | 52.5 |
| Lutein Ester* | 7.5mg Lutein | 50 |
| Vitamin C (Ascorbic Acid) | 100mg | 105 |
| Mixed Tocopherols 1000 iu/gm | 100mg | 149 |
| Selenium Yeast 1000 mcg/gm | 90mcg | 90 |
| Copper Gluconate to give | 3mg Cu | 22.26 |
| Zinc Gluconate to give | 15mg Zn | 117 |
| Manganese Gluconate to give | 4mg Mn | 36.4 |
| Vegetable Shortening | | 56 |
| Beeswax | | 23 |
| Lecithin | | 22 |
| Soya Bean Oil | | 75.563 |
| | | 800 |

| | | |
|---|---|---|
| *concentrated lutein esters with an E (1 %, 1cm) of 300 to 340 at 453 nm in chloroform - corresponds to a pure lutein content of 12 to 14.4%. | | |

One capsule per day is very suitable for long term administration and has in addition valuable antioxidant properties.

### Reference Example 8

### (Outside the Scope of the Invention)

A dry powder formula diet composition was prepared by mixing 150 mg of lutein ester per day with a Cambridge Diet (The Cambridge Diet is a Registered Trade Mark) product obtained from Cambridge Health Plan Ltd, Norwich, England under the product identification.

### References

Bone, R A and Landrum, J T (1984). Macular pigment in Henle fiber membranes: a model for Haidinger's brushes. Vision Res. 24, 103-108.
Bone, R A and Landrum, J T and Cains, A (1992). Optical density of the macular pigment *in vivo* and *in vitro.* Vision Res. 32, 105-110.
Bone, R A and Landrum, J T, Hime, G W, Cains, A and Zamor, J (1993). Stereochemistry of the human macular carotenoids. Invest. Ophthalmol. Vis. Sci. 34, 2033-2040.
Bone, R A and Landrum, J T, and Tarsis, S L (1985). Preliminary identification of the human macular pigment. Vision Res. 25, 1531-1535.
Bone, R A and Sparrock, J M B (1971). Comparison of macular pigment densities in human eyes. Vision Res. 11, 1057-1064.
Davson, H (1990). "Physiology of the Eye". Pergamon Press, Inc., New York.
Ditchburn, R W (1973). "Eye-Movements and Visual Perception". Clarendon Press, Oxford.
Eye Disease Case - Control Study Group (1993). Antioxidant status and neovascular age-related macular degeneration. Arch. Ophthalmol. 111, 104-109.
Feeney-Burns, L and Ellersieck, M R (1985). Age-related changes in the ultrastructure of Bruch's membrane. Am. I. Ophthalmol. 100, 686-697.
Gottsch, J D, Pou, S, Bynoa, L A and Rosen, G M (1990). Hematogenous photosensitization. A mechanism for development of age-related macular degeneration. Invest. Ophthalmol. Vis. Sci. 31, 1674-1692.
Guiliano, A R, Matzner, M B and Canfield, L M (1993). Assessing variability in quantitation of carotenoids in human plasma: variance component model. In "Methods in Enzymology" (L Packer, ed.), Vol 214, pp. 94-101. Academic Press, San Diego.
Guth, S L, Massof, R W and Benzschawel, T (1980). Vector model for normal and dichromatic colour vision. I. Opt. Soc. Am. 70, 197-212.
Hammond, B R, Jr., Fuld, K and Curran-Celentano, J (1995). Macular pigment density in monozygotic twins. Invest. Ophthalmol. Vis. Sci. 36, 2531-3541.
Handelmann, G J, Shen, B and Krinsky, N I (1992). High resolution analysis of carotenoids in human plasma by high-performance liquid chromatography. In "Methods in Enzymology" (L Packer, ed.), Vol 213, pp. 336-346. Academic Press, San Diego.
Kirshfeld, K (1982). Carotenoid pigments: their possible role in protecting against photooxidation in eyes and photoreceptor cells. Proc. R. Soc. Lond. B216, 71-85.
Landrum, J T, Bone, R A, Vidal, I, Menendez, E and Kilburn, M (1995). Macular pigment stereomers in individual eyes: a comparison between normals and those with age-related macular degeneration. Arch. Ophthalmol. 113, 1518-1523.
Mares-Perlman, J A, Brady, W E, Klein, R, Klein, B E K, Palta, M, Bowen, P and Stacewicz-Sapuntzakis, M (1994). Serum levels of carotenoids and tocopherols in people with age-related maculopathy. Invest. Ophthalmol. Vis. Sci. 35, (supply) 3455.
Pease, P L, Adem, A J and Nuccio, E (1987). Optical density of human macular pigment. Vision Res. 27, 705-710.
Reading, V M and Weale, R A (1974). Macular pigment and chromatic aberration. I. Opt. Soc. Am. 64, 231-234.
Ruttimann, A, Schiedt, T and Vecci, M (1983). Separation of (3R,3'R)-, (3R,3'S; meso)-(3S,3'S)-zeaxanthin, (3R,3'R,6'R)- (3R,3'S,6'S)- and (3S,3'S,6'S)-lutein via the dicarbamates of (S)-(-)-1-[1-naphthyl]lethylisocyanate. I. High. Res. Chrom. Commun. 6, 612-616.
Schalch, W (1992). Carotenoids in the retina. A review of their possible role in preventing or limiting damage caused by light and oxygen. EXS 62, 290-298.
Scheidt, K, Bischof, S and Glinz, E (1995). Example 5: Fish-isolation of astaxanthin and its metabolites from skin of Atlantic Salmon (Salmosalor). In "Carotenoids" (G Britton, S Liaaen-Jenson, H Pfander, eds.), pp. 243-252. Birkhauser Verlag, Basel.
Seddon, J M, Umed, A *et al* (1994). Dietary Carotenoids, Vitamins A, C and E and Advanced Age-Related Macular Degeneration. J.A.M.A., 272, 1413-1420.
Seddon, J M, Ajani, U A, Sperduto, R D *et al* (1995). Do antioxidants prevent or retard the onset of AMD? J. Am. Osteopath. Assoc. 95, 26.
Wald, G (1945). "Human vision and the spectrum". Nature (London). 101, 653-658.
Walls, G L (1967). "The Vertebrate Eye and its Adaptive Radiation". Hafner, New York.
Werner, J S, Donnelly, S K and Kliegl, R (1987). Ageing and human macular pigment density. Appended with translations from the work of Max Schutze and Ewald Hering. Vision Res. 27, 257-268.
Wooten, B R and Wald, G (1973). Colorvision mechanism in the peripheral retinas of normal and dichromatic observers. I. Gen. Physiol. 61, 125-145.
Wysecki, G and Stiles, W S (1982). "Quantitative Data and Formulae". Wiley, New York.

## Claims

1. A medicament comprising meso-zeaxanthin in combination with a pharmaceutical carrier or diluent.

2. A composition as claimed in claim 1, further comprising another biologically-active constituent.

3. A composition as claimed in claim 2, wherein the other biologically-active constituent is an anti-oxidant.

4. A composition as claimed in claim 3, wherein said anti-oxidant is another carotenoid or is vitamin A, vitamin C, vitamin E, selenium, copper, zinc, manganese or ubiquinone (Co enzyme Q10).

5. A composition as claimed in claim 4, wherein said other carotenoid is lutein; lycopene; or alpha, beta, gamma or delta carotene.

6. A composition as claimed in any preceding claim, in the form of a food supplement in which the meso-zeaxanthin is contained as a micronutrient.

7. A composition as claimed in any preceding claim and in unit dosage form containing 10 mg to 100mg of carotenoid(s) per dose.

8. A composition as claimed in claim 7, containing 20mg to 50mg of carotenoid(s) per dose.

9. A composition as claimed in claim 7, containing 30mg of carotenoid(s) per dose.

10. A composition as claimed in any preceding claim and in tablet, capsule, powder or solution suspension form.

11. A composition as claimed in any preceding claim and comprised of a mixture of lutein and meso-zeaxanthin comprising 10% to 90% by weight lutein and 90% to 10% by weight meso-zeaxanthin.

12. A composition as claimed in any preceding claim and in the form of a mixture comprising lutein and meso-zeaxanthin together with lecithin and soya bean oil.

13. Use of meso-zeaxanthin for the manufacture of a medicament for use in the treatment of macular depreciation of yellow pigment in the macula of an eye of a human patient.

14. A pharmaceutical treatment course package comprising means defining accessibly closed individual receptacles retaining respective dosage units of pharmaceutical composition comprising meso-zeaxanthin together with a carrier, said receptacles being arranged in the package in a first group of high dosage units and a second group of lower dosage units, the first group of receptacles numbering at least 14 and having in each one or more dosage units providing a total dosage of at least 10mg/receptacle of carotenoid(s).

15. A package as claimed in claim 14, wherein the second group of receptacles number at least 14.

16. A package as claimed in claim 14 or claim 15, wherein the second group of receptacles have in each one or more dosage units providing a total dosage of not more than 7.5mg/receptacle of carotenoids.

## Patentansprüche

1. Medikament, das meso-Zeaxanthin in Kombination mit einem pharmazeutischen Träger oder Verdünnungsmittel umfasst.

2. Zusammensetzung nach Anspruch 1, die außerdem einen weiteren biologisch aktiven Bestandteil umfasst.

3. Zusammensetzung nach Anspruch 2, worin der weitere biologisch aktive Bestandteil ein Antioxidans ist.

4. Zusammensetzung nach Anspruch 3, worin das Antioxidans ein weiteres Carotinoid ist oder Vitamin A, Vitamin C, Vitamin E, Selen, Kupfer, Zink, Mangan oder Ubichinon (Coenzym Q10) ist.

5. Zusammensetzung Anspruch 4, worin das weitere Carotinoid Lutein; Lycopin; oder Alpha-, Beta-, Gamma- oder Delta-Carotin ist.

6. Zusammensetzung nach einem der vorhergehenden Ansprüche, in Form eines Nahrungsergänzungsmittels, in dem das meso-Zeaxanthin als Mikronährstoff enthalten ist.

7. Zusammensetzung nach einem der vorhergehenden Ansprüche und in Einheitsdosisform, die 10 mg bis 100 mg Carotinoid(e) pro Dosis enthält.

8. Zusammensetzung nach Anspruch 7, die 20 mg bis 50 mg Carotinoid(e) pro Dosis enthält.

9. Zusammensetzung nach Anspruch 7, die 30 mg Carotinoid(e) pro Dosis enthält.

10. Zusammensetzung nach einem der vorhergehenden Ansprüche und in Tabletten-, Kapsel-, Pulver- oder Suspensionslösungsform.

11. Zusammensetzung nach einem der vorhergehenden Ansprüche und umfassend ein Gemisch aus Lutein und meso-Zeaxanthin, das 10 bis 90 Gew.% Lutein und 90 bis 10 Gew.-% meso-Zeaxanthin enthält.

12. Zusammensetzung nach einem der vorhergehenden Ansprüche und in Form eines Gemisches, das Lutein und meso-Zeaxanthin zusammen mit Lecithin und Sojabohnenöl enthält.

13. Verwendung von meso-Zeaxanthin zur Herstellung eines Medikaments zur Verwendung bei der Behandlung von makulärem Rückgang von gelbem Pigment in der Makula eines Auges eines menschlichen Patienten.

14. Pharmazeutische Kurpackung, die Mittel umfasst, welche in einer öffenbaren Weise verschlossene Einzelbehälter begrenzen, die jeweilige Dosierungseinheiten einer pharmazeutischen Zusammensetzung enthalten, welche meso-Zeaxanthin zusammen mit einem Träger umfasst, wobei diese Behälter in der Packung in einer ersten Gruppe hoher Dosierungseinheiten und einer zweiten Gruppe niedrigerer Dosierungseinheiten angeordnet sind, wobei die erste Gruppe von Behältern wenigstens 14 zählt und in jedem eine oder mehrere Dosierungseinheiten aufweist, die eine Gesamtdosis von wenigstens 10 mg Carotinoid(e)/Behälter bereitstellen.

15. Packung nach Anspruch 14, worin die zweite Gruppe von Behältern wenigstens 14 zählt.

16. Packung nach Anspruch 14 oder Anspruch 15, worin die zweite Gruppe von Behältern in jedem eine oder mehrere Dosierungseinheiten aufweist, die eine Gesamtdosis von nicht mehr als 7,5 mg Carotinoide/Behälter bereitstellen.

## Revendications

1. Médicament comprenant de la méso-zéaxanthine en combinaison avec un support ou un diluant pharmaceutique.

2. Composition selon la revendication 1, comprenant en outre un autre constituant biologiquement actif.

3. Composition selon la revendication 2, dans laquelle l'autre constituant biologiquement actif est un anti-oxydant.

4. Composition selon la revendication 3, dans laquelle ledit anti-oxydant est un autre caroténoïde ou est la vitamine A, la vitamine C, la vitamine E, le sélénium, le cuivre, le zinc, le manganèse ou l'ubiquinone (co-enzyme Q10).

5. Composition selon la revendication 4, dans laquelle l'autre caroténoïde est la lutéine ; le lycopène ; ou l'alpha, bêta, gamma ou delta carotène.

6. Composition selon l'une quelconque des revendications précédentes, sous la forme d'un complément alimentaire dans lequel la méso-zéaxanthine est contenue comme micronutriment.

7. Composition selon l'une quelconque des revendications précédentes, et sous une forme de dosage unitaire contenant 10 à 100 mg du ou des caroténoïde (s) par dose.

8. Composition selon la revendication 7, contenant 20 mg à 50 mg du ou des caroténoïde(s) par dose.

9. Composition selon la revendication 7, contenant 30 mg du ou des caroténoïde (s) par dose.

10. Composition selon l'une quelconque des revendications précédentes, et sous forme de comprimé, de capsule, de poudre ou de suspension pour solution.

11. Composition selon l'une quelconque des revendications précédentes et comprenant un mélange de lutéine et de méso-zéaxanthine comprenant 10 % à 90 % en poids de lutéine et 90 % à 10 % en poids de méso-zéaxanthine.

12. Composition selon l'une quelconque des revendications précédentes et sous la forme d'un mélange comprenant de la lutéine et de la méso-zéaxanthine avec de la lécithine et de l'huile de soja.

13. Utilisation de méso-zéaxanthine pour la fabrication d'un médicament à utiliser dans le traitement de la dépréciation maculaire du pigment jaune dans la macule d'un oeil d' un patient humain.

14. Emballage pour programme de traitement pharmaceutique comprenant un moyen définissant des réceptacles individuels fermés accessibles contenant des unités de dose respectives de composition pharmaceutique comprenant de la méso-zéaxanthine avec un support, lesdits réceptacles étant arrangés dans l'emballage en un premier groupe d'unités de dose élevée et un second groupe d'unités de dose plus faibles, le premier groupe de réceptacles en comptant au moins 14 et ayant dans chacun une unité de dose ou plus fournissant une dose totale d'au moins 10 mg de caroténoïde(s) par réceptacle.

15. Emballage selon la revendication 14, dans lequel le second groupe de réceptacles en compte au moins 14.

16. Emballage selon la revendication 14 ou la revendication 15, dans lequel les réceptacles du second groupe de réceptacles ont dans chacun une ou plusieurs unités de dose fournissant une dose totale ne dépassant pas 7,5 mg de caroténoïdes par réceptacle.
